# EUROPEAN PATENT APPLICATION

(11) **EP 3 246 703 A1**
(43) Date of publication of application: **22.11.2017**
(21) Application number: 16170645.2
(22) Date of filing: 20.05.2016
(51) Int. Cl.: G01N 33/50, G01N 33/543

(54) **METHOD AND KIT FOR CAPTURING EXTRACELLULAR VESICLES (EVS) ON A SOLID SURFACE**

(71) Applicant: Unicyte EV AG, 6370 Oberdorf (CH)
(72) Inventor: DEREGIBUS, Maria Chiara, 10132 Torino (IT); BUSSOLATI, Benedetta, 10126 Torino (IT); CAMUSSI, Giovanni, 10132 Torino (IT); DIMUCCIO, Veronica, 10128 Torino (IT)
(74) Representative: Comoglio, Elena

(57) **Abstract**

The invention relates to a method and a kit for capturing on a solid surface the extracellular vesicles (EVs) which are present in a biological fluid sample. The solid surface is coated with a polycationic substance, preferably a protamine salt such as protamine hydrochloride. The method and kit of the invention is useful for the detection and/or quantification of the extracellular vesicles (EVs) which are present in a biological fluid sample, particularly in a diagnostic context.

## Description

The present invention relates to methods and a kit for capturing extracellular vesicles (EVs) from a biological fluid sample and binding them to a solid substrate. The methods and kit of the invention are useful for the detection and quantification of EVs in biological fluid samples, particularly within the context of diagnostic applications.

Vesicular-mediated communication between cells appears critical in many biological processes. Small vesicles released from cells have recently emerged as important mediators of inter-cellular communication. These vesicles, that have been termed "extracellular vesicles (EVs)", are inclusive of exosomes released from the endosomal cell-membrane compartment and of microvesicles released from the cell surface by plasma membrane budding. The EV content of proteins, lipids and nucleic acids varies with the cell of origin and, after incorporation into recipient cells, they may transfer information which may change the phenotype and function of recipient cells.

Several studies have addressed the role of EVs in physiological and pathological conditions based on their biological activity and molecular constituents. Moreover, since EVs retain the signature of the cell of origin and are present in all body fluids, their potential use as diagnostics in different pathological conditions has been suggested.

A fundamental issue remains how to isolate EVs from cultured cells to study their biological functions or from biological fluids for diagnostic purposes. Since foetal bovine serum frequently used for cell culture is enriched in EVs, the *in vitro* experiments require the use of EVs-depleted serum. The isolation of EVs from body fluids, on the other hand, has to face the complexity due to the concomitant presence of EVs of different cellular origin. Therefore, in order to identify a potential biomarker, it is critical to discriminate cellular origin on the basis of EV molecular expression or content, by proteomic or genomic analysis. After removal of cell debris by centrifugation, three main methods are conventionally used for isolation of EVs, namely differential ultracentrifugation in the absence or presence of sucrose gradient, size exclusion chromatography and immune affinity. All these methods have some advantages, which are mainly related to the possibility to discriminate between different EV populations, and concerns, which are related to the risk of damaging vesicles during purification with loss of biological activity, to the need of a sufficiently large sample and to the efficiency of isolation.

Moreover, polymeric precipitation of EVs has been suggested as an alternative method mainly focused on the evaluation of RNA and protein content. The polymeric precipitation methods are based on the formation of a mesh-like net, which embeds EVs with a size ranging from 60 to 180 nm. Such methods may be applied either to culture media or to body fluids. In particular, polymeric precipitation methods may have the advantage for detection of biomarkers in vesicles derived from small biological samples.

Currently, the "gold standard" methods of EV purification are the differential ultracentrifugation or the density gradient ultracentrifugation. These methods, however, are influenced by several parameters difficult to standardize such as viscosity of solutions, rotor type, centrifugal radius and g force. In addition, the integrity of EVs after prolonged high speed ultracentrifugation may be damaged. In fact, membrane debris were observed by electron microscopy and difficulty in recovering RNA and exosomal proteins has been reported.

Several other approaches to EV purification have been investigated. The size exclusion chromatography may have an advantage on ultracentrifugation in maintaining EVs integrity, since with this method EVs are not subjected to shear stress. Filtration with membranes with appropriate pores is also an alternative, but it does not guarantee removal of several small contaminants and does not avoid loss of EVs by binding to membranes. Immunoaffinity purification may isolate specific exosome subtypes maintaining integrity of their cargo.

A limitation of most of these techniques is the efficiency in the recovery of sufficient amounts of EVs starting from small biological samples.

In order to overcome the drawbacks of the prior art, in particular the complexity and expensiveness of the above-mentioned prior art methods, their low efficiency in EV recovery, the possible presence of contaminants in the isolated EVs and the risk of damaging EV membranes, the present invention provides a method of capturing extracellular vesicles (EVs) from a biological fluid sample as defined in appended claim 1. The use of a solid surface coated with a polycationic substance for detecting or quantifying the extracellular vesicles (EVs) in a biological fluid sample also falls within the scope of the present invention.

Further features and advantages of the invention are defined in the dependent claims.

The method of capturing extracellular vesicles (EVs) according to the present invention results from the finding that EVs display a negative charge, which allows them to interact with positive-charged molecules.

Solid surfaces coated with protamine salt are known in the prior art. For example, Elisa plates coated with protamine sulfate are commercially available (Cosmo Bio Co., Ltd., Japan) and are used for binding DNA for quantification. However, to the inventors' knowledge, their use for capturing and detecting EVs has never been disclosed in the prior art. The inventors observerd improved results of EV precipitiation with protamine hydrochloride over other polycationic salts. A solid surface preferably selected from the group consisting of a microtiter plate, a column, a magnetic bead and a non-magnetic bead coated with protamine hydrochloride is therefore also an object of the invention.

Protamine coated on a solid surface was found to bind EVs from biological fluid samples without the need of further reagents or processing steps. Based on this finding, the present inventors also developed the immunoassay method for detecting or quantifying extracellular vesicles (EVs) in a biological fluid sample as defined in independent claim 9 and the related kit as defined in appended claim 16.

The methods and kit of the present invention have the merit of simplicity and avoid the requirement of expensive equipment and may be used for an efficient detection and quantification of EVs from small biological samples.

The method of capturing extracellular vesicles (EVs) from a biological fluid sample according to the present invention comprises the step of contacting the biological fluid sample with a polycationic substance coated on a solid surface, whereby the extracellular vesicles (EVs) which are present in the biological fluid sample are bound on the coated solid surface.

Any polycationic substance may be used in the method of the invention, on account of the negative charge of EVs. However, protamine, preferably in the form of a salt, is the preferred positive polycation because it is suitable for clinical applications. The most preferred form of protamine is protamine hydrochloride. Suitable alternatives to protamine are for example polylysine or cationic dextrans, preferably in the form of a salt such as hydrochloride.

The solid surface for use in the method of the invention is preferably selected from the group consisting of a microtiter plate, a column, a membrane, fibrous web, a magnetic bead and a non-magnetic bead. A microtiter plate, such as a 96 wells ELISA plate, is most preferred, particularly within the context of diagnostic applications.

The inventors employed the method of the invention to capture EVs from both biological fluids and from cell culture conditioned media. Accordingly, the method of the invention can be used for capturing EVs from any biological fluid or cell culture conditioned medium, such as for example from blood, serum, plasma, saliva, urine, cerebrospinal fluid, milk or from a cell culture conditioned medium, preferably an adult stem cell culture conditioned medium, more preferably a mesenchymal stem cell culture conditioned medium or a liver pluripotent progenitor cell culture conditioned medium.

As mentioned above, the EVs-capturing method according to the invention has the advantage that EVs are bound on the solid surface quite easily, without the need of further reagents or processing steps.

Therefore, the capturing method according to the invention can be conveniently used in an immunoassay method for detecting or quantifying extracellular vesicles (EVs) in a biological fluid sample.

The immunoassay method of the invention comprises the steps of:
(i) contacting the biological fluid sample with a solid surface coated with a polycationic substance, whereby the extracellular vesicles (EVs) which are present in the biological fluid sample are bound on the coated solid surface;
(ii) removing the unbound extracellular vesicles (EVs);
(iii) adding an antibody capable of binding to the extracellular vesicles (EVs) bound on the solid surface coated with the polycationic substance, thereby forming an immunocomplex;
(iv) detecting or quantifying the immunocomplex.

The polycationic substance and the solid surface which are preferably used in the immunoassay method of the invention are as defined above with reference to the capturing method.

According to a preferred embodiment of the immunoassay method of the invention, the antibody capable of binding to the extracellular vesicles (EVs) bound on the solid surface coated with the polycationic substance is labelled with a detectable label. For example, the antibody is a biotin-conjugated primary antibody and the immunocomplex resulting from the binding of the primary antibody to the EVs captured on the solid surface is detected by means of a Streptavidin HRP-conjugate. However, the person skilled in the art is able to select and use other labelling and detection means which are known per se in the art.

The scope of the invention also comprises a kit suitable for carrying out the immunoassay method of the invention, the kit comprising (i) a solid surface coated with a polycationic substance, as described above; and (ii) an antibody capable of binding to extracellular vesicles (EVs) bound on the solid surface coated with the polycationic substance, as described above. The kit of the invention may also comprise suitable detection means for detecting the immunocomplex resulting from the binding of the primary antibody to the EVs captured on the solid surface.

In another aspect of the invention the polycationic substance bound to the solid surface may serve as a ligand for adsorption of EV from biological liquids. In such cases the solid surface is preferably selected from the group of a column, a membrane, a fibrous web and adsorber beads. In such an application the polycationic substance bound to the solid surface is part of a filter or filter device.

A specific application of the method of the invention and of the filter and filter devices according to the invention relates to the removal of extracellular vesicles from patients' plasma or blood by adsorption.

The method of the invention for the adsorption of extracellular vesicles can be applied either ex vivo or in vivo, such as in a continuous extracorporeal circuit from blood or plasma in a way similar to common adsorption apheresis therapy. A specific application relates to the removal of extracellular vesicle associated with diseases mediated by extracellular vesicles, as described in WO2007/103572 or in EP2495025. Diseases mediated by extracellular vesicles comprise cancer, sepsis, SIRS, autoimmune diseases, neurodegenerative diseases, Parkinson's disease, Alzheimer's disease.

Continuous extracorporeal circuit for adsorption apheresis therapy are well known in the art. In continuous extracorporeal circuit care need to be taken that the patients blood does not coagulate in the extracorporeal circuit. Heparin is used commonly as an anti-coagulant. Since polycationic substance, in particular protamine, are known to bind heparin heparin-independent anti-coagulation methods are preferred. Local anti-coagulation of the extracorporeal circuit by citrate and an calcium as known from WO 91/06326 is a preferred method.

The method of adsorption can also be applied as a step in purification and concentration wherein the step of adsorption is followed by the elution of the extracellular vesicles from the filter for further use.

The following example is provided by way of illustration only and is not intended to limit the scope of the invention as determined by the appended claims.

### EXAMPLE

### ELISA test on protamine-coated plates.

The inventors set up an innovative ELISA test to quantify EVs in biological fluids and cell supernatant based on protamine binding.

### Methods

Generation of standard curves. EVs obtained by ultracentrifugation (1000.000 g, 2 hours) were loaded on protamine coated plates at decreasing concentration (starting from 2000 x 108 EVs). Wells were filled with 50 µl of sample and left overnight at 37°C, to dry. The next day, an anti-CD133 or an anti-CD24 antibodies (both biotin-conjugated) was added at a dilution of 1:50, followed by gentle rocking for 1h at room temperature (RT); a Streptavidin HRP-conjugated was used 1:30000 and incubated for 1h at RT. The absorbance was read in a spectro-photometer using 450nm as the primary wave length and 655 nm as reference wavelength.

### Results

### 1. Calibration lines of pooled EVs

Two markers of urinary vesicles CD133 and CD24 were used to generate standard curves. The analysis of the absorbance value for the CD133 expression correlated with the EV concentration as measured by NTA, with a R² value very close to 1.

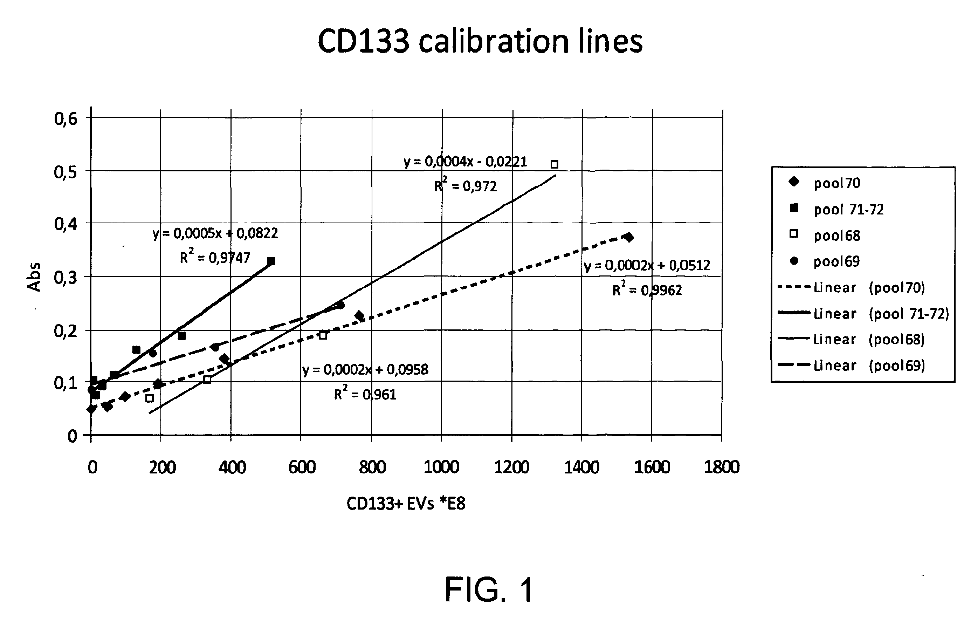

Figure 1 shows the CD133 calibration lines obtained with four different pools of EVs obtained by ultracentrifugation.

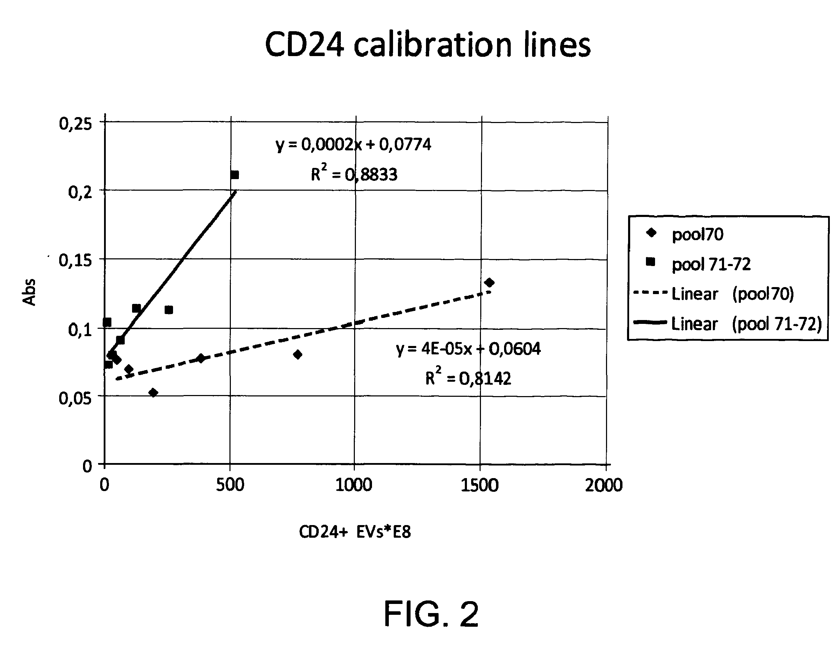

Similarly, the presence of CD24⁺ EVs correlated with the number of vesicles. Figure 2 shows the CD24 calibration lines obtained with two different pools of EVs obtained by ultracentrifugation.

### 2. Sample analysis

To evaluate the possibility to analyse the CD133 EVs in urine, a sample of undiluted total urine (50 µl) was tested in the ELISA assay (n=3). All samples gave a positive value in the range of the absorption line. We next compared the CD133⁺ EVs measured by cytofluorimetric analysis with the value measured with the ELISA assay. Table 1 shows the number of CD 133 EVs as result of the ELISA test and the number of EVs in the same volume as calculated by ultracentrifugation, followed by NTA and cytofluorimetric analysis.

**Table 1. Number of CD133 EVs*10⁸ as result of the ELISA test (calculated with the absorbance on the calibration line obtained by NTA and cytofluorimetric analysis) and the number of EVs in the same volume as calculated by ultracentrifugation, followed by NTA and cytofluorimetric analysis.**

| | ELISA | FACS + NTA |
|---|---|---|
| *1* | 395,25 | 1071,58 |
| *2* | 1025,75 | 1109,98 |
| *3* | 959 | 1103,11 |

Results show a good correlation.

## Claims

1. A method of capturing extracellular vesicles (EVs) from a biological fluid sample, the method comprising the step of contacting the biological fluid sample with a polycationic substance coated on a solid surface, whereby the extracellular vesicles (EVs) which are present in the biological fluid sample are bound on the coated solid surface.

2. The method according to claim 1, wherein the polycationic substance is selected from a protamine salt, a polylysine salt or a cationic dextran salt, wherein the salt is preferably a hydrochloride.

3. The method according to claim 1 or 2, wherein the solid surface is selected from the group consisting of a microtiter plate, a column, a membrane, fibrous web, a magnetic bead and a non-magnetic bead.

4. The method according to any of claims 1 to 3, wherein biological fluid is selected from the group consisting of blood, serum, plasma, saliva, urine, cerebrospinal fluid and the conditioned medium of a cell culture, wherein the cell culture is preferably an adult stem cell culture, more preferably a mesenchymal stem cell culture or a liver pluripotent progenitor cell culture.

5. The use of a solid surface coated with a polycationic substance for detecting or quantifying the extracellular vesicles (EVs) in a biological fluid sample.

6. The use according to claim 5, wherein the polycationic substance is selected from a protamine salt, a polylysine salt or a cationic dextran salt, wherein the salt is preferably a hydrochloride.

7. The use according to claim 5 or 6, wherein the solid surface is selected from the group consisting of a microtiter plate, a column, a membrane, fibrous web, a magnetic bead and a non-magnetic bead.

8. The use according to any of claims 5 to 7, wherein biological fluid is selected from the group consisting of blood, serum, plasma, saliva, urine, cerebrospinal fluid, milk and the conditioned medium of a cell culture, wherein the cell culture is preferably an adult stem cell culture, more preferably a mesenchymal stem cell culture or a liver pluripotent progenitor cell culture.

9. An immunoassay method of detecting or quantifying extracellular vesicles (EVs) in a biological fluid sample, comprising the steps of:
(i) contacting the biological fluid sample with a solid surface coated with a polycationic substance, whereby the extracellular vesicles (EVs) which are present in the biological fluid sample are bound on the coated solid surface;
(ii) removing the unbound extracellular vesicles (EVs);
(iii) adding an antibody capable of binding to the extracellular vesicles (EVs) bound on the solid surface coated with the polycationic substance, thereby forming an immunocomplex;
(iv) detecting or quantifying the immunocomplex.

10. The method according to claim 9, wherein the polycationic substance is selected from a protamine salt, a polylysine salt or a cationic dextran salt, wherein the salt is preferably a hydrochloride.

11. The method according to claim 9 or 10, wherein the solid surface is selected from the group consisting of a microtiter plate, a magnetic bead and a non-magnetic bead.

12. The method according to any of claims 9 to 11, wherein biological fluid is selected from the group consisting of blood, serum, plasma, saliva, urine, cerebrospinal fluid, milk and the conditioned medium of a cell culture, wherein the cell culture is preferably an adult stem cell culture, more preferably a mesenchymal stem cell culture or a liver pluripotent progenitor cell culture.

13. The method according to any of claims 9 to 12, wherein the antibody capable of binding to the extracellular vesicles (EVs) bound on the solid surface coated with the polycationic substance is labeled with a detectable label.

14. The method according to any of claims 9 to 13, wherein the antibody capable of binding to the extracellular vesicles (EVs) bound on the solid surface coated with the polycationic substance is an anti-CD133 antibody or an anti-CD 24 antibody.

15. The method according to any of claims 9 to 14, for the *in vitro* diagnosis of a disease.

16. A kit for detecting or quantifying extracellular vesicles (EVs) in a biological fluid sample, comprising:
(i) a solid surface coated with a polycationic substance; and
(ii) an antibody capable of binding to extracellular vesicles (EVs) bound on the solid surface coated with the polycationic substance.

17. The kit according to claim 16, wherein the the polycationic substance is selected from a protamine salt, a polylysine salt or a cationic dextran salt, wherein the salt is preferably a hydrochloride.

18. The kit according to claim 16 or 17, wherein the solid surface is selected from the group consisting of a microtiter plate, a magnetic bead and a non-magnetic bead.

19. The kit according to any of claims 16 to 18, wherein biological fluid is selected from the group consisting of blood, serum, plasma, saliva, urine, cerebrospinal fluid, milk and the conditioned medium of a cell culture, wherein the cell culture is preferably an adult stem cell culture, more preferably a mesenchymal stem cell culture or a liver pluripotent progenitor cell culture.

20. The kit according to any of claims 16 to 19, wherein the antibody capable of binding to the extracellular vesicles (EVs) bound on the solid surface coated with the polycationic substance is labeled with a detectable label.

21. The kit according to any of claims 16 to 20, wherein the antibody capable of binding to the extracellular vesicles (EVs) bound on the solid surface coated with the polycationic substance is an anti-CD133 antibody or an anti-CD 24 antibody.

22. The kit according to any of claims 16 to 21, for use in the *in vitro* diagnosis of a disease.

23. Solid surface preferably selected from the group consisting of a microtiter plate, a column, a magnetic bead and a non-magnetic bead coated with protamine hydrochloride.

24. A polycationic substance bound to a solid surface selected from the group of a column, a membrane, fibrous web for use in the treatment of an extracellular vesicle mediated disease wherein the treatment comprises the removal of extracellular vesicles from blood or blood components in an extracorporeal circuit.

25. A polycationic substance bound to a solid surface for use in the treatment of an extracellular vesicle mediated disease according to claim 24 wherein the extracellular vesicle mediated disease is selected from the group of cancer, sepsis, SIRS, autoimmune diseases, neurodegenerative diseases, Parkinson's disease, Alzheimer's disease.
